(19) Europäisches Patentamt
European Patent Office
Office européen des brevets

(11) EP 3 263 018 A1

(12) EUROPEAN PATENT APPLICATION

(43) Date of publication:
03.01.2018 Bulletin 2018/01

(51) Int Cl.:
A61B 5/00 (2006.01)     A61B 8/12 (2006.01)
A61B 5/02 (2006.01)     A61B 5/021 (2006.01)
A61B 5/0215 (2006.01)

(21) Application number: 16176925.2

(22) Date of filing: 29.06.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Koninklijke Philips N.V.
5656 AE Eindhoven (NL)

(72) Inventor: The designation of the inventor has not
yet been filed

(74) Representative: de Haan, Poul Erik et al
Philips International B.V.
Philips Intellectual Property & Standards
High Tech Campus 5
5656 AE Eindhoven (NL)

(54) DEVICES AND METHODS FOR STRATIFICATION OF PATIENTS FOR RENAL DENERVATION BASED ON INTRAVASCULAR PRESSURE AND WALL THICKNESS MEASUREMENTS

(57) Devices and methods for pulse wave velocity determination are disclosed. The apparatus includes an intravascular device (110) that can be positioned within a vessel (80). The intravascular device includes a flexible elongate member (170) having a proximal portion and a distal portion. A pressure sensor (204) can be coupled to the distal portion of the flexible elongate member. The pressure sensor can monitor pressure within the vessel. At least one imaging element (202) can be coupled to the distal portion of the flexible elongate member. The imaging element can monitor the wall thickness of the vessel. A processing system (130) in communication with the intravascular device can control the monitoring of the pressure and the monitoring of the wall thickness of the vessel. The processing system can receive pressure data and wall thickness data and determine a pulse wave velocity of fluid within the vessel.

Fig. 1a

EP 3 263 018 A1

**Description**

TECHNICAL FIELD OF THE INVENTION

Embodiments of the present disclosure relate generally to the field of medical devices and, more particularly, to devices, systems, and methods for patient stratification for renal denervation.

BACKGROUND OF THE INVENTION

Hypertension and its associated conditions, chronic heart failure (CHF) and chronic renal failure (CRF), constitute a significant and growing global health concern. Current therapies for these conditions span the gamut covering non-pharmacological, pharmacological, surgical, and implanted device-based approaches. Despite the vast array of therapeutic options, the control of blood pressure and the efforts to prevent the progression of heart failure and chronic kidney disease remain unsatisfactory.

Blood pressure is controlled by a complex interaction of electrical, mechanical, and hormonal forces in the body. The main electrical component of blood pressure control is the sympathetic nervous system (SNS), a part of the body's autonomic nervous system, which operates without conscious control. The sympathetic nervous system connects the brain, the heart, the kidneys, and the peripheral blood vessels, each of which plays an important role in the regulation of the body's blood pressure. The brain plays primarily an electrical role, processing inputs and sending signals to the rest of the SNS. The heart plays a largely mechanical role, raising blood pressure by beating faster and harder, and lowering blood pressure by beating slower and less forcefully. The blood vessels also play a mechanical role, influencing blood pressure by either dilating (to lower blood pressure) or constricting (to raise blood pressure).

The kidneys play a central electrical, mechanical and hormonal role in the control of blood pressure. The kidneys affect blood pressure by signaling the need for increased or lowered pressure through the SNS (electrical), by filtering blood and controlling the amount of fluid in the body (mechanical), and by releasing key hormones that influence the activities of the heart and blood vessels to maintain cardiovascular homeostasis

(hormonal). The kidneys send and receive electrical signals from the SNS and thereby affect the other organs related to blood pressure control. They receive SNS signals primarily from the brain, which partially control the mechanical and hormonal functions of the kidneys. At the same time, the kidneys also send signals to the rest of the SNS, which can boost the level of sympathetic activation of all the other organs in the system, effectively amplifying electrical signals in the system and the corresponding blood pressure effects. From the mechanical perspective, the kidneys are responsible for controlling the amount of water and sodium in the blood, directly affecting the amount of fluid within the circulatory system. If the kidneys allow the body to retain too much fluid, the added fluid volume raises blood pressure. Lastly, the kidneys produce blood pressure regulating hormones including renin, an enzyme that activates a cascade of events through the renin-angiotensin-aldosterone system (RAAS). This cascade, which includes vasoconstriction, elevated heart rate, and fluid retention, can be triggered by sympathetic stimulation. The RAAS operates normally in non-hypertensive patients but can become overactive among hypertensive patients. The kidney also produces cytokines and other neurohormones in response to elevated sympathetic activation that can be toxic to other tissues, particularly the blood vessels, heart, and kidney. As such, overactive sympathetic stimulation of the kidneys may be responsible for much of the organ damage caused by chronic high blood pressure.

Thus, overactive sympathetic stimulation of the kidneys plays a significant role in the progression of hypertension, CHF, CRF, and other cardio-renal diseases. Heart failure and hypertensive conditions often result in abnormally high sympathetic activation of the kidneys, creating a vicious cycle of cardiovascular injury. An increase in renal sympathetic nerve activity leads to the decreased removal of water and sodium from the body, as well as increased secretion of renin, which leads to vasoconstriction of blood vessels supplying the kidneys. Vasoconstriction of the renal vasculature causes decreased renal blood flow, which causes the kidneys to send afferent SNS signals to the brain, triggering peripheral vasoconstriction and increasing a patient's hypertension. Reduction of sympathetic renal nerve activity, e.g., via renal neuromodulation or denervation of the renal nerve plexus, may reverse these processes.

Efforts to control the consequences of renal sympathetic activity have included the administration of medications such as centrally acting sympatholytic drugs, angiotensin converting enzyme inhibitors and receptor blockers (intended to block the RAAS), diuretics (intended to counter the renal sympathetic mediated retention of sodium and water), and beta-blockers (intended to reduce renin release). The current pharmacological strategies have significant limitations, including limited efficacy, compliance issues, and side effects.

As noted, renal denervation is a treatment option for resistant hypertension. However, the efficacy of renal denervation can be very variable between patients. Recent, studies indicate that the velocity of the pressure/flow pulse (pulse wave velocity or PWV) inside the main renal artery can be indicative of the outcome of renal denervation. The PWV in patient with resistant hypertension can be very high (e.g., more than 20 m/s), which can make it difficult to determine the PWV in the relatively short renal arteries (e.g., 5-8 cm in length).

**EP 3 263 018 A1**

While the existing treatments have been generally adequate for their intended purposes, they have not been entirely satisfactory in all respects. The medical devices, systems, and associated methods of the present disclosure overcome one or more of the shortcomings of the prior art.

SUMMARY OF THE INVENTION

The present disclosure describes calculation of a physiological quantity known as a pulse wave velocity (PWV). The PWV represents the velocity of the pressure and flow waves that propagate through the blood vessels of a patient as a result of the heart pumping. Recent studies have indicated that the PWV within the renal artery, which is an artery that supplies blood to the kidney, is indicative of whether a therapeutic known as renal denervation will be successful in the patient. Renal denervation is often used to treat hypertension. As described in more detail herein, PWV can be calculated based on monitoring vessel wall thickness using an imaging element and measuring pressure using a pressure sensor. The imaging element and the pressure sensor can be attached to an intravascular device positioned within the vessel. The pulse wave velocity of fluid within the vessel can be calculated using a mathematical relationship of the pressure, and the vessel wall thickness. The calculated PWV for the patient can then be used to determine whether the patient is good candidate for treatment. For example, the PWV measurement result can be used to perform patient stratification for the renal denervation, before performing the treatment, by predicting the efficacy of renal denervation based on PWV.

In one exemplary embodiment, the present disclosure describes an apparatus for pulse wave velocity (PWV) determination in a vessel that comprises an intravascular device that can be positioned within the vessel. The intravascular device can include a flexible elongate member that can have a proximal portion and a distal portion. A pressure sensor can be coupled to the distal portion of the flexible elongate member. The pressure sensor can monitor a pressure within the vessel. At least one imaging element can be coupled to the distal portion of the flexible elongate member. The at least one imaging element can monitor a wall thickness of the vessel. The apparatus can include a processor that can be in communication with the intravascular device. The processor can control the monitoring of the pressure within the vessel. The processor can also control the monitoring of the wall thickness of the vessel by the at least one imaging element. The processor can receive pressure data associated with the monitoring of the pressure within the vessel and wall thickness data associated with monitoring of the wall thickness of the vessel. The processor can determine a pulse wave velocity of fluid based on the pressure data and the wall thickness data.

In some instances, pulse wave velocity is determined by the equation: $\sqrt{\dfrac{dP}{2\rho} \cdot \dfrac{h}{-dh}}$ (also shown below as equation (4)). In the equation, $h$ is a thickness of the vessel wall and $dh$ is the change in the vessel wall thickness as a result of a pressure change $dP$. Additionally, $\rho$ is a density of a fluid within the vessel.

As an example, the vessel wall thickness $h$ can be averaged in a cross section of the vessel. For example, the cross section of the vessel at the location of the imaging element can be measured and the vessel wall thickness around the boundary of the cross section can be averaged. In another example, the vessel wall thickness $h$ can be averaged in multiple cross sections around the imaging element. In an embodiment, the wall thickness may be determined in only one segment of the vessel wall.

Additionally, in the equation, the vessel wall thickness $h$ can be determined with an imaging element, based on e.g. optical coherence tomography (OCT). The pressure data that is used for PWV determination can be determined with the pressure sensor. Since this can be a local measurement in the vessel, it can be exceptionally suitable for PWV determination in the renal arteries for stratification of patients for renal artery denervation, but also suitable for use in other vessels.

In another exemplary embodiment, the present disclosure describes an apparatus for pulse wave velocity (PWV) determination in a vessel that comprises an intravascular device that can include a flexible elongate member that can have a proximal portion and a distal portion. A pressure sensor can be coupled to the distal portion of the flexible elongate member and can monitor a pressure within the vessel. The apparatus can include at least one imaging element that can monitor a wall thickness of the vessel. Alternatively, the imaging element can be coupled to an intravascular probe separate from the intravascular device that has the pressure sensor. The apparatus can also include a processor that can be in communication with the pressure sensor and the at least one imaging element. The processor can control the monitoring of the pressure within the vessel and the monitoring of the wall thickness of the vessel by the at least one imaging element. The processor can synchronize the monitoring of the pressure within the vessel by the pressure sensor and the monitoring of the wall thickness of the vessel by the at least one imaging element. The processor can receive pressure data associated with the monitoring of the pressure within the vessel and wall thickness data associated with monitoring of the wall thickness of the vessel. The processor can determine a pulse wave velocity of fluid based on the pressure data and the wall thickness data.

3

In another exemplary embodiment, the present disclosure describes a method for determining pulse wave velocity (PWV) in a vessel. The method comprises monitoring a pressure within the vessel with a pressure sensor positioned within the vessel and monitoring a wall thickness of the vessel. The method also includes receiving pressure data associated with the monitoring of the pressure within the vessel and wall thickness data associated with monitoring of the wall thickness of the vessel. The method further includes determining the pulse wave velocity of a fluid within the vessel based on the pressure data within the vessel and the wall thickness data of the vessel.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

BRIEF DESCRIPTION OF THE DRAWINGS

The accompanying drawings illustrate embodiments of the devices and methods disclosed herein and together with the description, serve to explain the principles of the present disclosure.

FIG. 1 a is a schematic illustration of a system including an intravascular device having a pressure sensor and an imaging element.

FIG. 1b is a schematic illustration of a system including an intravascular device having a pressure sensor and a separate intravascular device having an imaging element.

FIG. 2 is a schematic diagram illustrating an intravascular device positioned within the renal anatomy.

FIG. 3 is a schematic diagram illustrating a cross-sectional view of a segment of a renal artery.

FIG. 4a is a schematic diagram illustrating a perspective view of a portion of the renal nerve plexus overlying a segment of a renal artery.

FIG. 4b is a schematic diagram illustrating a perspective view of an example portion of the renal nerve plexus overlying a segment of a renal artery.

FIG. 5a is a graph of pressure measurements associated with pulse waves travelling through a vessel.

FIG. 5b shows graphs of pressure measurements associated with pulse waves travelling through a vessel at two different locations within the vessel.

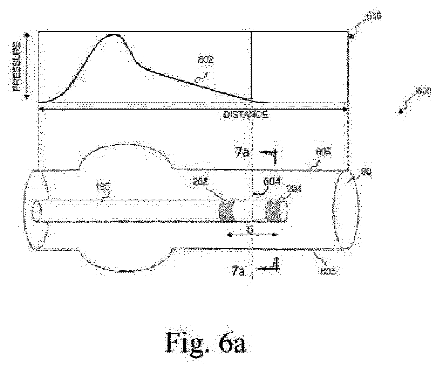

Collectively, FIGS. 6a-7c illustrate aspects of a vessel as a pulse wave is travelling through the vessel.

FIG. 6a is a schematic diagram illustrating an intravascular device within a vessel at a first stage of a pulse wave.

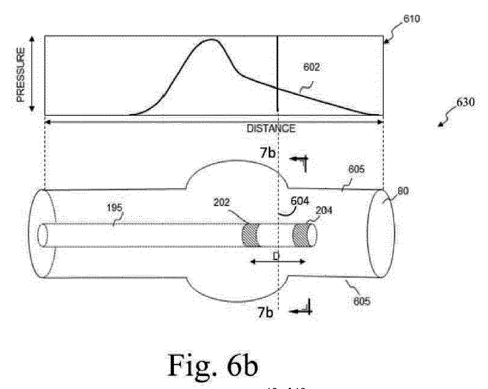

FIG. 6b is a schematic diagram illustrating an intravascular device within a vessel similar to that of Fig. 6a, but at a second stage of the pulse wave.

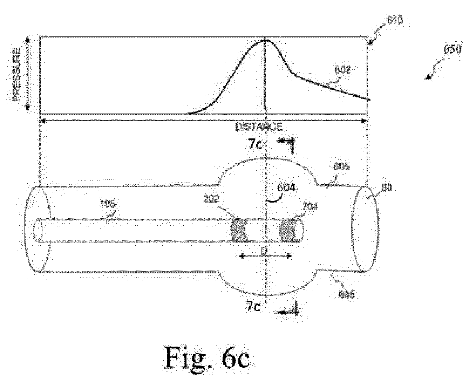

FIG. 6c is a schematic diagram illustrating an intravascular device within a vessel similar to that of FIGS. 6a and 6b, but at a third stage of the pulse wave.

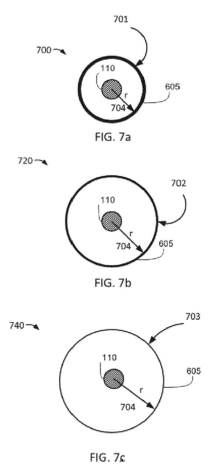

FIG. 7a is a schematic diagram illustrating a cross-sectional view of the vessel associated with the first stage of the pulse wave shown in Fig. 6a.

FIG. 7b is a schematic diagram illustrating a cross-sectional view of the vessel associated with the second stage of the pulse wave shown in Fig. 6b.

FIG. 7c is a schematic diagram illustrating a cross-sectional view of the vessel associated with the third stage of the pulse wave shown in Fig. 6c.

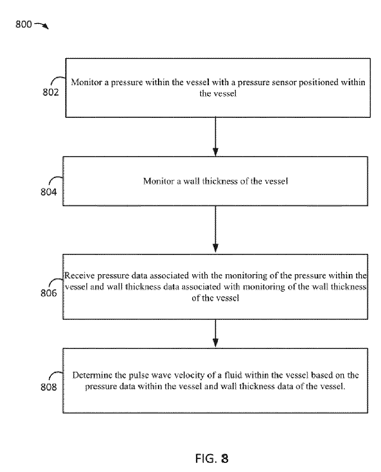

FIG. 8 is a schematic flowchart illustrating a method of determining pulse wave velocity in a vessel.

DETAILED DESCRIPTION OF THE EMBODIMENTS

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. In addition, dimensions provided herein are for specific examples and it is contemplated that different sizes, dimensions, and/or ratios may be utilized to implement the concepts of the present disclosure. For the sake of brevity, however, the numerous iterations of these combinations will not be described separately. For simplicity, in some instances the same reference numbers are used throughout the drawings to refer to the same or like parts.

The present disclosure relates generally to devices, systems, and methods for determining/measuring pulse wave velocity in a main renal artery prior to a renal denervation treatment. The velocity of the pressure/flow pulse (pulse wave velocity or PWV) inside the main renal artery can be predictive of the outcome of renal denervation. The PWV can be very high in resistive hypertension patients, which makes it very difficult to perform an accurate measurement of PWV in the relatively short renal arteries. Multiple pressure sensing devices positioned within a vessel (e.g., renal vessel 80) can be used to determine the PWV in the vessel. However, the sampling frequency of the pressure sensors can be a limiting factor when using this method in determining PWV in short vessels, such as the renal arteries. Another way to determine the PWV is by utilizing the "water hammer" equation to calculate the PWV from simultaneous pressure and flow velocity measurements inside the vessel during a reflection free period (e.g., early systole):

$$PWV = \frac{1}{\rho}\frac{dP}{dU} \qquad (1)$$

Or, alternatively, in case this reflection free period cannot be used the following relation can be used that determines the PWV by summation over the whole cardiac cycle:

$$PWV = \frac{1}{\rho}\sqrt{\frac{\sum dP^2}{\sum dU^2}} \qquad (2)$$

with p being the blood density and P and U the pressure and velocity, respectively. The disadvantage of that method is that it requires intravascular flow velocity measurement, which can be challenging to perform due to orientation/location dependence of the sensor. Additionally, being on a guide-wire the pressure and flow velocity sensor are not located at exact the same location on the guide-wire, which decreases the accuracy of the PWV determination. An alternative way to determine the PWV is by simultaneous measurement of the pressure and visualizing the extension of the arterial wall due to the wave. The PWV can then be determined by the Bramwell-Hill equation:

$$PWV = \sqrt{\frac{dP}{\rho}\frac{A}{dA}} \qquad (3)$$

with $A$ being the cross-sectional area of the lumen and $dA$ the change in cross-sectional area as a result of a pressure change $dP$. Alternatively, PWV is given by the Moens-Korteweg equation:

$$PWV = \sqrt{\frac{E \cdot h}{2r \cdot \rho}}$$

where E is the Young's modulus, $r$ is the vessel radius, and $h$ is the wall thickness. The Bramwell-Hill equation, in fact derived from the Moens-Korteweg equation, can be rewritten in terms of the lumen radius $r$ as:

$$PWV = \sqrt{\frac{dP}{2\rho}\frac{r}{dr}}$$

Assuming that the vessel wall is thin and incompressible, with a wall thickness $h$ much smaller than the lumen radius $r$, the change in lumen radius can be expressed in terms of the wall thickness change $dh$ as

$$dr = -\frac{r}{h}dh$$

The minus sign indicates that the wall thickness decreases if the lumen radius increases and vice versa. We can use this expression to calculate PWV as a function of the wall thickness as:

$$PWV = \sqrt{\frac{dP}{2\rho} \cdot \frac{h}{-dh}} \qquad (4)$$

In some embodiments, the vessel wall thickness can be estimated in synchronization with the heartbeat. For example, the baseline measurement can be performed at the end of diastole, just before the next cardiac pulse appears, and the second measurement can be performed at the peak systolic pressure, when the pressure is at a maximum. A method to obtain appropriate sampling can involve a sampling of the imaging element signal with a pulse repetition frequency of up to about 100 Hz. For example, the maximum and minimum of both the wall thickness and the pressure can be determined within one heart cycle, and *dP* and *dh* may be taken as the difference between the maximum and minimum of the pressure and wall thickness, respectively. This approach yields one PWV measurement per heartbeat. For an increased accuracy, the PWV estimate may be averaged over a number of cardiac cycles.

As noted, renal denervation is a treatment option for resistant hypertension. Recent studies indicate that the velocity of the pressure/flow pulse (pulse wave velocity or PWV) inside the main renal artery pre-treatment can be predictive of the outcome of renal denervation treatment. In some instances, embodiments of the present disclosure are configured to perform pulse wave velocity measurements of the renal artery for stratification of patients for renal artery denervation. Renal sympathetic activity may worsen symptoms of hypertension, heart failure, and/or chronic renal failure. In particular, hypertension has been linked to increased sympathetic nervous system activity stimulated through any of four mechanisms, namely (1) increased vascular resistance, (2) increased cardiac rate, stroke volume and output, (3) vascular muscle defects, and/or (4) sodium retention and renin release by the kidney. As to this fourth mechanism in particular, stimulation of the renal sympathetic nervous system can affect renal function and maintenance of homeostasis. For example, an increase in efferent renal sympathetic nerve activity may cause increased renal vascular resistance, renin release, and sodium retention, all of which exacerbate hypertension.

As an example, thermal neuromodulation by either intravascular heating or cooling may decrease renal sympathetic activity by disabling the efferent and/or afferent sympathetic nerve fibers that surround the renal arteries and innervate the kidneys through renal denervation, which involves selectively disabling renal nerves within the sympathetic nervous system (SNS) to create at least a partial conduction block within the SNS.

Several forms of renal injury or stress may induce activation of the renal afferent signals (e.g., from the kidney to the brain or the other kidney). For example, renal ischemia, a reduction in stroke volume or renal blood flow, may trigger activation of renal afferent nerve activity. Increased renal afferent nerve activity results in increased systemic sympathetic activation and peripheral vasoconstriction (narrowing) of blood vessels. Increased vasoconstriction results in increased resistance of blood vessels, which results in hypertension. Increased renal efferent nerve activity (e.g., from the brain to the kidney) results in further increased afferent renal nerve activity and activation of the RAAS cascade, inducing increased secretion of renin, sodium retention, fluid retention, and reduced renal blood flow through vasoconstriction. The RAAS cascade also contributes to systemic vasoconstriction of blood vessels, thereby exacerbating hypertension. In addition, hypertension often leads to vasoconstriction and atherosclerotic narrowing of blood vessels supplying the kidneys, which causes renal hypoperfusion and triggers increased renal afferent nerve activity. In combination this cycle of factors results in fluid retention and increased workload on the heart, thus contributing to the further cardiovascular and cardio-renal deterioration of the patient.

Renal denervation, which affects both the electrical signals going into the kidneys (efferent sympathetic activity) and the electrical signals emanating from them (afferent sympathetic activity) can impact the mechanical and hormonal activities of the kidneys themselves, as well as the electrical activation of the rest of the SNS. Blocking efferent sympathetic activity to the kidney may alleviate hypertension and related cardiovascular diseases by reversing fluid and salt retention (augmenting natriuresis and diuresis), thereby lowering the fluid volume and mechanical load on the heart, and reducing inappropriate renin release, thereby halting the deleterious hormonal RAAS cascade.

By blocking afferent sympathetic activity from the kidney to the brain, renal denervation may lower the level of activation of the whole SNS. Thus, renal denervation may also decrease the electrical stimulation of other members of the sympathetic nervous system, such as the heart and blood vessels, thereby causing additional anti-hypertensive effects. In addition, blocking renal nerves may also have beneficial effects on organs damaged by chronic sympathetic over-activity, because it may lower the level of cytokines and hormones that may be harmful to the blood vessels, kidney, and heart.

Furthermore, because renal denervation reduces overactive SNS activity, it may be valuable in the treatment of several other medical conditions related to hypertension. These conditions, which are characterized by increased SNS activity, include left ventricular hypertrophy, chronic renal disease, chronic heart failure, insulin resistance (diabetes and metabolic syndrome), cardio-renal syndrome, osteoporosis, and sudden cardiac death. For example, other benefits of renal denervation may theoretically include: reduction of insulin resistance, reduction of central sleep apnea, improvements in perfusion to exercising muscle in heart failure, reduction of left ventricular hypertrophy, reduction of ventricular rates in patients with atrial fibrillation, abrogation of lethal arrhythmias, and slowing of the deterioration of renal function in chronic kidney disease. Moreover, chronic elevation of renal sympathetic tone in various disease states that exist with or without hypertension may play a role in the development of overt renal failure and end-stage renal disease. Because the reduction of afferent renal sympathetic signals contributes to the reduction of systemic sympathetic stimulation, renal denervation may also benefit other organs innervated by sympathetic nerves. Thus, renal denervation may also alleviate various medical conditions, even those not directly associated with hypertension.

In some embodiments, the PWV may be predictive of the outcome of renal denervation in treating resistive hypertension. As described herein, the computing device can output the calculated PWV to a display. A clinician may make therapeutic and/or diagnostic decisions, taking the PWV into consideration, such as whether to recommend the patient for a renal denervation procedure. In some instances, the computer system can determine and output a therapy recommendation or a likelihood-of-success prediction to the display, based on the PWV and/or other patient data. That is, the computer system may utilize the PWV to identify which patients are more likely and/or less likely to benefit from renal denervation.

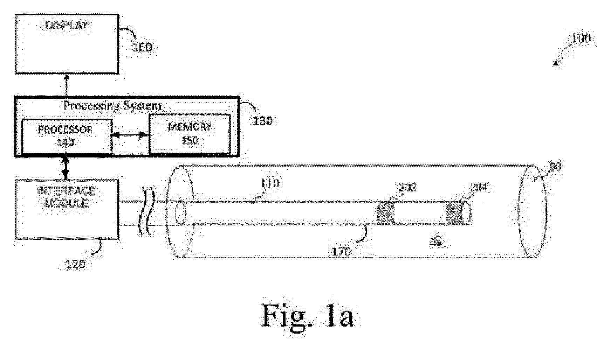

Fig. 1a is a diagrammatic schematic view of an exemplary system 100 according to some embodiments of the present disclosure. The system 100, which may be referred to as a stratification system, may be configured to perform pulse wave velocity (PWV) determination in a vessel 80 (e.g., artery, vein, etc.), for patient stratification for treatment purposes. For example, the PWV determination in the renal arteries may be utilized to determine whether a patient is suitable for renal artery denervation. The system 100 may include an intravascular device 110 that may be positioned within the vessel 80, an interface module 120, a processing system 130 having at least one processor 140 and at least one memory 150, and a display 160.

In some embodiments, the system 100 may be configured to perform pulse wave velocity (PWV) determination in a vessel 80 within a body portion. The intravascular system 100 may be referred to as a stratification system in that the PWV may be used for patient stratification for treatment purposes. For example, the PWV determination in the renal arteries may be utilized to determine whether a patient is suitable for renal artery denervation. Based on the PWV determination, the intravascular system 100 maybe used to classify one or more patients into groups respectively associated with varying degrees of predicted therapeutic benefit of renal denervation. Any suitable number of groups or categories are contemplated. For example, the groups may include groups respectively for those patients with low, moderate, and/or high likelihood of therapeutic benefit from renal denervation, based on the PWV. Based on the stratification or classification, the system 100 can recommend the degree to which one or more patients are suitable candidates for renal denervation.

The vessel 80 may represent fluid-filled or surrounded structures, both natural and man-made. The vessel 80 may be within a body of a patient. The vessel 80 may be a blood vessel, as an artery or a vein of a patient's vascular system, including cardiac vasculature, peripheral vasculature, neural vasculature, renal vasculature, and/or or any other suitable lumen inside the body. For example, the intravascular device 110 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the heart, chambers or other parts of the heart, and/or other systems of the body. In addition to natural structures, the device intravascular 110 may be used to examine man-made structures such as, but without limitation, heart valves, stents, shunts, filters and other devices. Walls of the vessel 80 define a lumen 82 through which fluid flows within the vessel 80.

The vessel 80 may be located within a body portion. When the vessel 80 is the renal artery, the patient body portion may include the abdomen, lumbar region, and/or thoracic region. In some examples, the vessel 80 may be located within any portion of the patient body, including the head, neck, chest, abdomen, arms, groin, legs, etc.

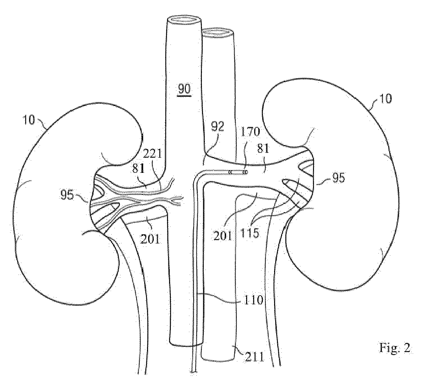

In some embodiments, the intravascular device 110 may include a flexible elongate member 170 such as a catheter, guide wire, or guide catheter, or other long, thin, long, flexible structure that may be inserted into a vessel 80 of a patient. In some embodiments, the vessel 80 is a renal artery 81 as shown in Fig. 2. While the illustrated embodiments of the intravascular device 110 of the present disclosure have a cylindrical profile with a circular cross-sectional profile that defines an outer diameter of the intravascular device 110, in other instances, all or a portion of the intravascular device may have other geometric cross-sectional profiles (e.g., oval, rectangular, square, elliptical, etc.) or non-geometric cross-sectional profiles. In some embodiments, the intravascular device 110 may or may not include a lumen extending along all or a portion of its length for receiving and/or guiding other instruments. If the intravascular device 110 includes a lumen, the lumen may be centered or offset with respect to the cross-sectional profile of the intravascular device 110.

The intravascular device 110, or the various components thereof, may be manufactured from a variety of materials, including, by way of non-limiting example, plastics, polytetrafluoroethylene (PTFE), polyether block amide (PEBAX), thermoplastic, polyimide, silicone, elastomer, metals, such as stainless steel, titanium, shape-memory alloys such as Nitinol, and/or other biologically compatible materials. In addition, the intravascular device may be manufactured in a variety of lengths, diameters, dimensions, and shapes, including a catheter, guide wire, a combination of catheter and guide wire, *etc.* For example, in some embodiments the flexible elongate member 170 may be manufactured to have length ranging from approximately 115 cm - 155 cm. In one particular embodiment, the flexible elongate member 170 may be manufactured to have length of approximately 135 cm. In some embodiments, the flexible elongate member 170 may be manufactured to have an outer transverse dimension or diameter ranging from about 0.35 mm - 2.67 mm (1 Fr - 8 Fr). In one embodiment, the flexible elongate member 170 may be manufactured to have a transverse dimension of 2 mm (6 Fr) or less, thereby permitting the intravascular device 110 to be configured for insertion into the renal vasculature of a patient. These examples are provided for illustrative purposes only, and are not intended to be limiting. In some examples, the intravascular device 195 is sized and shaped such that it can be moved inside the vasculature (or other internal lumen(s)) of a patient such that the pressure and wall thickness of a vessel can be monitored from within the vessel.

In some embodiments, the intravascular device 110 includes a sensor 202 and a sensor 204 disposed along the length of the flexible elongate member 170. The sensors 202, 204 may be configured to collect data about conditions within the vessel 80, and in particular, identify changes in the vessel wall of the vessel 80.

In an example, the sensor 202 includes an optical imaging element (e.g., a mirror, lens, prism, etc. and/or combinations thereof) in communication with coherent light source (*e.g.,* a laser source) and a light detector such that optical coherence tomography imaging can be used to determine the wall thickness of the vessel. In some implementations, the sensor 202 is an optical acoustic transducer.

OCT systems operate in either the time domain or frequency (high definition) domain. In time-domain OCT, an interference spectrum is obtained by moving a scanning optic, such as a reference minor, longitudinally to change the reference path and match multiple optical paths due to reflections of the light within the sample. The signal giving the reflectivity is sampled over time, and light traveling at a specific distance creates interference in the detector. Moving the scanning mechanism laterally (or rotationally) across the sample produces reflectance distributions of the sample (i.e., an imaging data set) from which two-dimensional and three-dimensional images can be produced. In frequency domain OCT, a light source capable of emitting a range of optical frequencies passes through an interferometer, where the interferometer combines the light returned from a sample with a reference beam of light from the same source, and the intensity of the combined light is recorded as a function of optical frequency to form an interference spectrum. A Fourier transform of the interference spectrum provides the reflectance distribution along the depth within the sample. Alternatively, in swept-source OCT, the interference spectrum is recorded by using a source with adjustable optical frequency, with the optical frequency of the source swept through a range of optical frequencies, and recording the interfered light intensity as a function of time during the sweep. Time- and frequency-domain systems can further vary based upon the optical layout of the systems: common beam path systems and differential beam path systems. A common beam path system sends all produced light through a single optical fiber to generate a reference signal and a sample signal whereas a differential beam path system splits the produced light such that a portion of the light is directed to the sample and the other portion is directed to a reference surface. OCT systems and methods are generally described in Castella et al., U.S. Pat. No. 8,108,030, Milner et al., U.S. Patent Application Publication No. 2011/0152771, Condit et al., U.S. Patent Application Publication No. 2010/0220334, Castella et al., U.S. Patent Application Publication No. 2009/0043191, Milner et al., U.S. Patent Application Publication No. 2008/0291463, and Kemp, N., U.S. Patent Application Publication No. 2008/0180683, U.S. Pat. No. 5,321,501, U.S. Pat. No. 7,999,938; U.S. Pat. No. 7,995,210, U.S. Pat. No. 7,787,127, U.S. Pat. No. 7,783,337; U.S. Pat. No. 6,134,003; and U.S. Pat. No. 6,421,164, the content of each of which is incorporated by reference in their entireties.

Generally, the sensor 202 (and/or other similar sensors) can be used to obtain an imaging data from the vessel, from which the processing system 130 generates an intravascular image. The processing system 130 can determine one or more measurement values associated with the vessel, such as cross-sectional area, radius, diameter, wall thickness, and/or distance from the sensor to the vessel wall from the intravascular image.

The intravascular device 110 can also include a pressure sensor 204 coupled to the distal portion of the flexible elongate member 170. The sensor 204 may be configured to collect data about conditions within the vessel 80, and in particular, monitor a pressure within the vessel 80. Furthermore, the sensor 204 may periodically measure the pressure of fluid (*e.g.,* blood) at the location of the sensor 204 inside the vessel 80. In an example, the sensor 204 is a capacitive pressure sensor, or in particular, capacitive MEMS pressure sensor. In another example, sensor 204 is a piezo-resistive pressure sensor. In yet another example, sensor 204 is an optical pressure sensor. In some instances, the sensor 204 includes components similar or identical to those found in commercially available pressure monitoring elements such as the PrimeWire PRESTIGE® pressure guide wire, the PrimeWire® pressure guide wire, and the Combo Wire® XT pressure and flow guide wire, each available from Volcano Corporation. In some embodiments, blood pressure measurements may be used to identify pulse waves passing through the vessel.

As shown in Fig. 6a, the sensors 202, 204 may be disposed a first distance D apart. In some embodiments, the distance D is fixed distance from 0.5 to 10 cm. In some embodiments, the fixed distance is smaller than 0.5 cm. In some examples, the two sensors are integrated and the distance is zero. In some embodiments, the distance D is within 0.5 to 2 cm. The distance D1 may be used in the calculation of Pulse Wave Velocity (PWV).

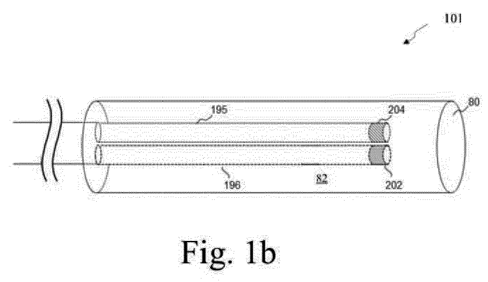

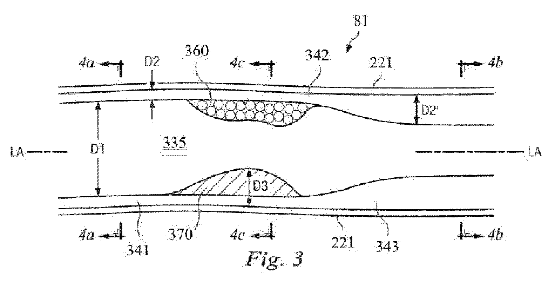

The sensors 202, 204 may be contained within the body of the intravascular device 110. The sensors 202, 204 may be disposed circumferentially around a distal portion of the intravascular device 110. In other embodiments, the sensors 202, 204 are disposed linearly along the intravascular device 110. The sensors 202, 204 may include one or more transducer elements. The sensor 202 and/or the sensor 204 may be movable along a length of the intravascular device 110 and/or fixed in a stationary position along the length of the intravascular device 110. The sensors 202, 204 may be part of a planar or otherwise suitably-shaped array of sensors of the intravascular device 110. In some embodiments, the outer diameter of the flexible elongate member 170 is equal to or larger than the outer diameter of the sensors 202, 204. In some embodiments, the outer diameter of the flexible elongate member 110 and sensors 202, 204 are equal to or less than about 1 mm, which may help to minimize the effect of the intravascular device 110 on pressure wave measurements within the vessel 80. In some examples, the vessel 80 in FIG. 1a, FIG. 1b, FIG. 3a, and FIG. 3b is a renal vessel consistent with the vessels 81 of Figure 2 and the renal artery has a cross section with an equivalent circular diameter of approximately 5 mm, a 1 mm outer diameter of the intravascular device 110 may obstruct less than 4% of the vessel.

The processing system 130 may be in communication with the intravascular device 110. For example, the processing system 130 may communicate with the intravascular device 110, including the sensor 202 and/or the sensor 204, through an interface module 120. The processor 140 may include any number of processors and may send commands and receive responses from the intravascular device 110. In some implementations, the processor 140 controls the monitoring of the pressure within the vessel 80 by the pressure sensor 204 and/or controls the monitoring of the thickness of the wall of the vessel 80 by the imaging element 202. In particular, the processor 140 may be configured to trigger the activation of the sensors 202, 204 to obtain data at specific times. Data from the sensors 202, 204 may be received by a processor of the processing system 130. In other embodiments, the processor 140 is physically separated from the intravascular device 110 but in communication with the intravascular device 110 (*e.g.,* via wireless communications). In some embodiments, the processor is configured to control the sensors 202, 204.

The processing system 130 can also receive the pressure data associated with the monitoring of the pressure within the vessel 80 and receive the imaging data associated with monitoring of the wall thickness of the vessel 80. In some embodiments, the interface module 120 can receive both the pressure signals corresponding to pressure monitoring from the pressure sensor 204 and the imaging signals corresponding to the wall thickness monitoring from the imaging element 202. In other instances, separate interface modules may be provided for the pressure and imaging data. The interface module 120 can process, preprocess, and/or sample the received pressure sensor signal and/or the received imaging element signal. The interface module 120 can transfer the pressure data and wall thickness data to the processing system 130. In some embodiments, received data is stored in the memory 150 of the processing system 130.

The processor 140 may include an integrated circuit with power, input, and output pins capable of performing logic functions such as commanding the sensors and receiving and processing data. The processor 140 may include any one or more of a microprocessor, a controller, a digital signal processor (DSP), an application specific integrated circuit (ASIC), a field-programmable gate array (FPGA), or equivalent discrete or integrated logic circuitry. In some examples, processor 140 may include multiple components, such as any combination of one or more microprocessors, one or more controllers, one or more DSPs, one or more ASICs, or one or more FPGAs, as well as other discrete or integrated logic circuitry. The functions attributed to processor 140 herein may be embodied as software, firmware, hardware or any combination thereof.

The processing system 130 may include one or more processors or programmable processor units running programmable code instructions for implementing the pulse wave velocity determination methods described herein, among other functions. The processing system 130 may be integrated within a computer and/or other types of processorbased devices. For example, the processing system 130 may be part of a console, tablet, laptop, handheld device, or other controller used to generate control signals to control or direct the operation of the intravascular device 110. In some embodiments, a user may program or direct the operation of the intravascular device 110 and/or control aspects of the display 160. In some embodiments, the processing system 130 maybe in direct communication with the intravascular device 110 (e.g., without an interface module 120), including via wired and/or wireless communication techniques.

Moreover, in some embodiments, the interface module 120 and processing system 130 are collocated and/or part of the same system, unit, chassis, or module. Together the interface module 120 and processing system 130 assemble, process, and render the sensor data for display as an image on a display 160. For example, in various embodiments, the interface module 120 and/or processing system 130 generate control signals to configure the sensors 202, 204, generate signals to activate the sensors 202, 204, perform calculations of sensor data, perform amplification, filtering, and/or aggregating of sensor data, and format the sensor data as an image for display. The allocation of these tasks and others may be distributed in various ways between the interface module 120 and processing system 130. In particular, the processing system 130 may use the received sensor data to calculate a pulse wave velocity of the fluid (*e.g.,* blood) inside the vessel 80. The interface module 120 can include circuitry configured to facilitate transmission of control signals from the processing system 130 to the intravascular device 110, as well as the transmission of pressure data from the intravascular device 110 to the processing system 130. In some embodiments, the interface module 120 can provide power to the sensors 202, 204. In some embodiments, the interface module can perform signal conditioning and/or pre-processing of the pressure data prior to transmission to the processing system 130.

The processing system 130 may be in communication with an electrocardiograph (ECG) console configured to obtain ECG data from electrodes positioned on the patient. ECG signals are representative of electrical activity of the heart and can be used to identify the patient's cardiac cycle and/or portions thereof. In some instances, the processing system 130 can utilize different formula to calculate PWV based on whether the pressure data obtained by the intravascular device 110 is obtained over an entire cardiac cycle and/or a portion thereof. The ECG data can be used to identify the beginning and ending of the previous, current, and next cardiac cycle(s), the beginning and ending of systole, the beginning and ending of diastole, among other portions of the cardiac cycle. In some examples, one or more identifiable feature of the ECG signal (including without limitation, the start of a P-wave, the peak of a P-wave, the end of a P-wave, a PR interval, a PR segment, the beginning of a QRS complex, the start of an R-wave, the peak of an R-wave, the end of an R-wave, the end of a QRS complex (J-point), an ST segment, the start of a T-wave, the peak of a T-wave, and the end of a T-wave) can utilized to select relevant portions of the cardiac cycle. The ECG console may include features similar or identical to those found in commercially available ECG elements such as the PageWriter cardiograph system available from Koninklijke Philips N.V.

Various peripheral devices may enable or improve input and output functionality of the processing system 130. Such peripheral devices may include, but are not necessarily limited to, standard input devices (such as a mouse, joystick, keyboard, *etc.*), standard output devices (such as a printer, speakers, a projector, graphical display screens, *etc.*), a CD-ROM drive, a flash drive, a network connection, and electrical connections between the processing system 130 and other components of the system 100. By way of non-limiting example, the processing system 130 may manipulate signals from the intravascular device 110 to generate an image on the display 160 representative of the acquired pressure data, imaging data, PWV calculations, and/or combinations thereof. Such peripheral devices may also be used for downloading software containing processor instructions to enable general operation of the intravascular device 110 and/or the processing system 130, and for downloading software implemented programs to perform operations to control, for example, the operation of any auxiliary devices coupled to the intravascular device 110. In some embodiments, the processing system 130 may include a plurality of processing units employed in a wide range of centralized or remotely distributed data processing schemes.

The memory 150 may be a semiconductor memory such as, for example, read-only memory, a random access memory, a FRAM, or a NAND flash memory. The memory 150 may interface with the processor 140 and associated processors such that the processor 140 may write to and read from the memory 150. For example, the processor 140 may be configured to receive data from the intravascular device 110 and/or the interface module 120 and write that data to the memory 150. In this manner, a series of data readings may be stored in the memory 150. The processor 140 may be capable of performing other basic memory functions, such as erasing or overwriting the memory 150, detecting when the memory 150 is full, and other common functions associated with managing semiconductor memory.

The processing system 130 can use the received pressure data and the wall thickness data to determine (e.g., calculate) a pulse wave velocity of the fluid (e.g., blood) inside the vessel. In some embodiments, the vessel is an artery. In an example, the vessel is a renal artery. In some embodiments, the processing system 130 can use the equation (4) to calculate the pulse wave velocity. In an example, the processor 140 can synchronize the received pressure data and wall thickness data and use the synchronized data to calculate the pulse wave velocity of equation (4). As shown above, equation (4) uses the vessel wall thickness $h$, a change in the vessel wall thickness $dh$, a change in pressure $dP$, as well as the density of a fluid within the vessel $\rho$.

In an example, the pressure sensor and imaging element signals can be synchronized by the processor 140. The interface module 120 can include a timer and the processor 140 by communicating to the interface module 120 can synchronize the timer of the interface module 120 with the processor timer. Additionally, the interface module 120 can do the sampling of the signals received from imaging element 202 and the pressure sensor 204 and can include a time stamp to the sampled data and then send the time-stamped sampled data to the processor 140 such that the pressure data associated with the monitoring of the pressure within the vessel and wall thickness data associated with monitoring of the wall thickness of the vessel that is received by the processor 140 is time-stamped and the processor 140 can synchronized the data based on the received time stamps.

Alternatively, instead of the interface module 120, the imaging element 202 and the pressure sensor 204 can perform the sampling and send the sampled data to the interface module 120. The imaging element 202 and the pressure sensor 204 can include a timer and the processor 140 by communicating to the imaging element 202 and the pressure sensor 204 can synchronize them with the processor timer. Thus, the data received from imaging element 202 and the pressure sensor 204 can include a time stamp and the interface module 120 can use the time stamps to synchronize the received data and then send it the processor 140. In another example, the interface module 120 can send the time-stamped data received from the imaging element 202 and the pressure sensor 204 to the processor 140 and the processor 140 can synchronized the data based on the received time stamps.

In some embodiments, as described herein, one or more feature of the ECG signal can be used to trigger data collection by the sensors 202, 204 in a synchronized manner.

In some embodiments, the imaging element 202 may not be part of the intravascular device 110. For example, the imaging element 202 may be coupled to a separate intravascular device or may be part of an external imaging device.

Referring to FIG. 1b, a schematic illustration of a system 101 including an intravascular device having a pressure sensor and a separate intravascular device having an imaging element are shown. The system 101 includes a first intravascular device 195 and a second intravascular device 196 inside a vessel 80. The first intravascular device 195 includes a pressure sensor 204 and the second intravascular device 196 includes an imaging element 202. The system 101, which may be referred to as a stratification system, may be configured to perform pulse wave velocity (PWV) determination in a vessel (*e.g.*, artery, vein, etc.), for patient stratification for treatment purposes. The system 101 can be coupled through the interface module 120, to the processing system 130 having the processor 140 and the memory 150, shown in FIG. 1a, and can perform PWV determination. For example, the PWV determination in the renal arteries can be performed the may be utilized to determine whether a patient is suitable for renal artery denervation. Generally, the pressure sensor 204 may be coupled to one of a guide wire or a catheter, and the imaging element 202 may be coupled to the other of the guide wire or the catheter. In some instances, the first intravascular device 194 may be a guide wire, and the second intravascular device 196 may be a catheter. The first and second intravascular devices 194, 196 can be positioned side by side within the vessel 80 in some embodiments. In some embodiments, a guide wire can at least partially extend through and be positioned within a lumen of the catheter such that the catheter and guide wire are coaxial.

FIG. 2 illustrates the intravascular device 110 in positioned within the human renal anatomy. The human renal anatomy includes kidneys 10 that are supplied with oxygenated blood by right and left renal arteries 81, which branch off an abdominal aorta 90 at the renal ostia 92 to enter the hilum 95 of the kidney 10. The abdominal aorta 90 connects the renal arteries 81 to the heart. Deoxygenated blood flows from the kidneys 10 to the heart via renal veins 201 and an inferior vena cava 211. Specifically, the intravascular device 110 is shown extending through the abdominal aorta and into the left renal artery 81. In alternate embodiments, the catheter may be sized and configured to travel through the inferior renal vessels 115 as well.

Left and right renal plexi or nerves 221 surround the left and right renal arteries 81, respectively. Anatomically, the renal nerve 221 forms one or more plexi within the adventitial tissue surrounding the renal artery 81. For the purpose of this disclosure, the renal nerve is defined as any individual nerve or plexus of nerves and ganglia that conducts a nerve signal to and/or from the kidney 10 and is anatomically located on the surface of the renal artery 81, parts of the abdominal aorta 90 where the renal artery 81 branches off the aorta 90, and/or on inferior branches of the renal artery 81. Nerve fibers contributing to the plexi arise from the celiac ganglion, the lowest splanchnic nerve, the corticorenal ganglion, and the aortic plexus. The renal nerves 221 extend in intimate association with the respective renal arteries into the substance of the respective kidneys 10. The nerves are distributed with branches of the renal artery to vessels of the kidney 10, the glomeruli, and the tubules. Each renal nerve 221 generally enters each respective kidney 10 in the area of the hilum 95 of the kidney, but may enter the kidney 10 in any location, including the location where the renal artery 81, or a branch of the renal artery 81, enters the kidney 10.

Proper renal function is essential to maintenance of cardiovascular homeostasis so as to avoid hypertensive conditions. Excretion of sodium is key to maintaining appropriate extracellular fluid volume and blood volume, and ultimately controlling the effects of these volumes on arterial pressure. Under steady-state conditions, arterial pressure rises to that pressure level which results in a balance between urinary output and water and sodium intake. If abnormal kidney function causes excessive renal sodium and water retention, as occurs with sympathetic overstimulation of the kidneys through the renal nerves 221, arterial pressure will increase to a level to maintain sodium output equal to intake. In hypertensive patients, the balance between sodium intake and output is achieved at the expense of an elevated arterial pressure in part as a result of the sympathetic stimulation of the kidneys through the renal nerves 221. Renal denervation may help alleviate the symptoms and sequelae of hypertension by blocking or suppressing the efferent and afferent sympathetic activity of the kidneys 10.

In some embodiments, the vessel 80 in FIG. 1a and FIG. 1b is a renal vessel consistent with the vessels 81 of Figure 2 and the pulse wave velocity is determined in the renal artery. The processing system 130 may determine the pulse wave velocity (PWV) in the renal artery. The processing system 130 may determine a renal denervation therapy recommendation based on the pulse wave velocity in a renal artery. For example, patients that are more likely or less likely to benefit therapeutically from renal denervation may be selected based on the PWV. In that regard, based at least on the PWV of blood in the renal vessel, the processing system 130 can perform patient stratification for renal denervation.

FIG. 3 illustrates a segment of the renal artery 81 in greater detail, showing various intraluminal characteristics and intra-to-extraluminal distances that may be present within a single vessel. In particular, the renal artery 81 includes a lumen 335 that extends lengthwise through the renal artery along a longitudinal axis LA. The lumen 335 is a tubelike passage that allows the flow of oxygenated blood from the abdominal aorta to the kidney. The sympathetic renal nerves 221 can extend within the adventitia surrounding the renal artery 81, and include both the efferent (conducting away from the central nervous system) and afferent (conducting toward the central nervous system) renal nerves.

The renal artery 81 includes a first portion 341 having an essentially healthy luminal diameter D1 and an intra-to-extraluminal distance D2, a second portion 342 having a narrowed and irregular lumen and an enlarged intra-to-extraluminal distance D3 due to atherosclerotic changes in the form of plaques 360, 370, and a third portion 343 having a narrowed lumen and an enlarged intra-to-extraluminal distance D2' due to a thickened arterial wall. Thus, the intraluminal contour of a vessel, for example, the renal artery 81, may be greatly varied along the length of the vessel.

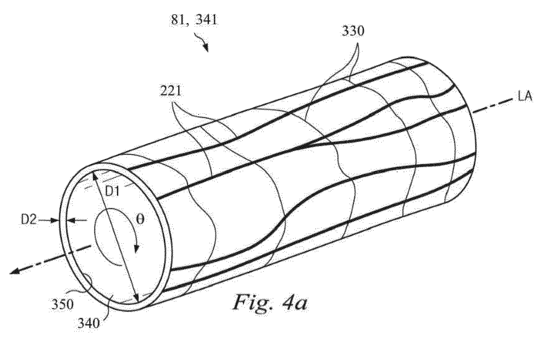

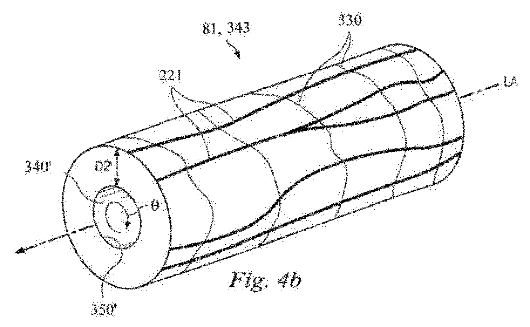

FIGS. 4a and 4b illustrate the portions 341, 343, 342, respectively, of the renal artery 81 in perspective view, showing the sympathetic renal nerves 221 that line the renal artery 81. FIG. 4a illustrates the portion 341 of the renal artery 81 including the renal nerves 221, which are shown schematically as a branching network attached to the external surface of the renal artery 81. The renal nerves 221 can extend lengthwise along the longitudinal axis LA of renal artery 81. In the case of hypertension, the sympathetic nerves that run from the spinal cord to the kidneys 10 signal the body to produce norepinephrine, which leads to a cascade of signals ultimately causing a rise in blood pressure. Renal denervation of the renal nerves 221 removes or diminishes this response and facilitates a return to normal blood pressure.

The renal artery 81 has smooth muscle cells 330 that surround the arterial circumference and spiral around the angular axis $\theta$ of the artery. The smooth muscle cells 330 of the renal artery 81 have a longer dimension extending transverse (i.e., non-parallel) to the longitudinal axis LA of the renal artery 81. The misalignment of the lengthwise dimensions of the renal nerves 221 and the smooth muscle cells 330 is defined as "cellular misalignment." This cellular misalignment of the renal nerves 221 and the smooth muscle cells 330 may be exploited to selectively affect renal nerve cells with a reduced effect on smooth muscle cells.

In FIG. 4a, the first portion 341 of the renal artery 81 includes a lumen 340 that extends lengthwise through the renal artery along the longitudinal axis LA. In some examples, the lumen 340 is a cylindrical passage that allows the flow of oxygenated blood from the abdominal aorta to the kidney. The lumen 340 includes a luminal wall 350 that forms the blood-contacting surface of the renal artery 81. The distance D1 corresponds to the luminal diameter of lumen 340 and defines the diameter or perimeter of the blood flow lumen. A distance D2, corresponding to the wall thickness, exists between the luminal wall 350 and the renal nerves 221. The relatively healthy renal artery 81 may have an almost uniform distance D2 or wall thickness with respect to the lumen 340. The relatively healthy renal artery 81 may decrease substantially regularly in cross-sectional area and volume per unit length, from a proximal portion near the aorta to a distal portion near the kidney.

FIG. 4b illustrates the third portion 343 of the renal artery 81 including a lumen 340' that extends lengthwise through the renal artery along the longitudinal axis LA. The lumen 340' includes a luminal wall 350' which forms the blood-contacting surface of the renal artery 81. In some patients, the smooth muscle wall of the renal artery is thicker than in other patients, and consequently, as illustrated in FIG. 3, the lumen of the third portion 343 of the renal artery 81 possesses a smaller diameter relative to the renal arteries of other patients. In some examples, the lumen 340', which is smaller in diameter and cross-sectional area than the lumen 340 pictured in FIG. 4a, is a cylindrical passage that allows the flow of oxygenated blood from the abdominal aorta to the kidney. A distance D2' exists between the luminal wall 350' and the renal nerves 221 that is greater than the distance D2 pictured in FIG. 4a.

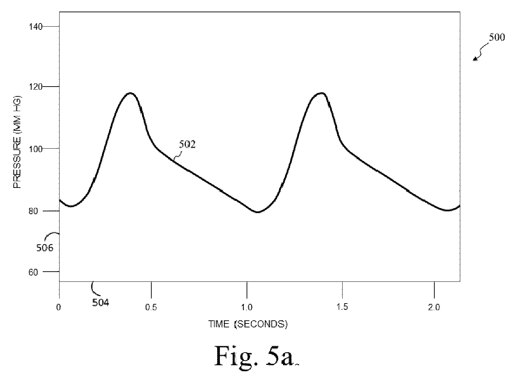

FIG. 5a is a graph 500 of pressure measurements associated with pulse waves travelling through a vessel. The graph 500 shows a pressure curve 502 of a fluid, e.g., blood, travelling through a vessel. The horizontal axis 504 can represent time and the vertical axis 506 can represent the fluid pressure in millimeters of mercury. For example, the graph 500 depicts two complete pulses, each one taking about 1 second (corresponding to a heart rate of approximately 60 beats per minute). As an example, the curve 502 of FIG. 5a can represent the pressure wave as a function of time at a specific point, *e.g.,* the location of the pressure sensor 204 inside the vessel 80.

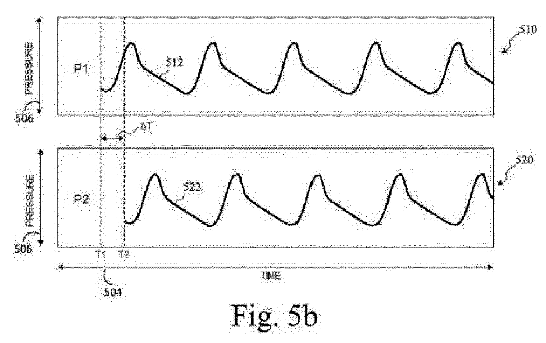

FIG. 5b shows graphs of pressure measurements associated with pulse waves travelling through a vessel at two different locations within the vessel. The graph 510 shows a pressure curve 512 of a fluid, e.g., blood, travelling through a vessel at a first location within the vessel, while graph 520 shows a pressure curve 522 of the fluid at a second location within the vessel. In some instances, the second location is distal or downstream of the fluid flow from the first location. The horizontal axes 504 of the graphs 510 and 520 can represent time and the vertical axes 506 can represent the fluid pressure in millimeter of mercury. The pressure curves 512 and 522 illustrate the significant change in pressure between the two locations at any given time. Thus, it can be important to keep the pressure sensor 204 and imaging element 202 in close proximity to each other such that they monitor the same location inside the vessel and/or make a high resolution sampling of the pressure sensor and imaging element signals such that the resulting pressure data and wall thickness data can be synchronized. In some examples, in a flexible vessel the increase/decrease of the pressure causes a corresponding expansion/contraction the vessel that can be monitored by the associated increase/decrease in the cross-sectional area of the vessel 80.

In some embodiments, the pressure can be monitored within 1 cm of the monitoring of the cross-sectional area of the vessel. Referring back to FIG. 1a, the pressure sensor 204 can be positioned within 1 cm of the imaging element 202 along a length of the flexible elongate member 170 of the intravascular device 110. In an example, this limitation can be incorporated into the design specification of the intravascular device 110. Also, referring back to FIG. 1b, the pressure sensor 204 can be positioned within 1 cm of the imaging element 202 when the intravascular device 195 and the intravascular device 196 are inserted into the vessel 80. In an example, the pressure sensor 204 and the imaging element 202 can be mechanically aligned within the 1 cm using guidewires to adjust the insertion length of intravascular device 195 and the intravascular device 196. Also, the imaging element 202 can be used to find the distance between the imaging element 202 and pressure sensor 204 and the guidewires can be used to adjust/align the distance to within the 1 cm and to keep the imaging element 202 and pressure sensor 204 aligned. Additionally, a separate system such as a control module executing on the processor 140 of FIG. 1a can control the guidewire coupled to the intravascular device 110 and the position of the imaging element and to keep them aligned. Alternatively, referring back to FIGS. 1b, an imaging system separate from the imaging element 202 can monitor the pressure sensor and/or the location of the imaging element 202 and through the processor 140 can keep the imaging element 202 and pressure sensor 204 aligned.

In some embodiments, assuming a wave speed of 8 m/s in the vessel, and an artery diameter of 5 mm, and a pulse pressure of 40 mmHg, and a wall thickness of 0.3 mm, then *dh* can be determined as 10 $\mu$m; if the PWV is 10 m/s, dh is 6.5 $\mu$m. The spatial resolution of the wall thickness assessment therefore needs to be able to detect wall changes of 3.5 $\mu$m. In some examples, the measurements can be determined with OCT. FIGS. 6a-c illustrate aspects of a vessel as a pulse wave is travelling through the vessel. FIGS. 6a-c are schematic examples of a vessel including the intravascular device 195 when a pulse wave is travelling through the vessel according to one embodiment of the present disclosure. As noted above, the vessel of FIGS. 6a-c is flexible and thus as the pressure moves through the vessel its cross-sectional area changes. The graph 610 shows the pressure wave as a function of position at different instances of time in the vessel 80. As shown in the figure, the vessel 80 can expand and its cross-sectional area can increase or its wall thickness can decrease (stretch) as the pressure pulse increases. In particular, the dashed line 604 shows a specific cross section being measured at different instances of time. FIG. 6a is a schematic diagram illustrating the intravascular device 195 within the vessel 80 at a first stage of a pulse wave. At this stage the pressure wave is at its minimum and the vessel boundary is not expanded (e.g., vessel wall 605 is not stretched). FIG. 6b is a schematic diagram 630 illustrating the intravascular device 195 within a vessel 80 similar to that of Fig. 6a, but at a second stage when the pressure wave is midway between minimum and the peak of the pulse wave and the vessel boundary is somewhat expanded and the vessel wall 605 is some stretched. FIG. 6c is a schematic diagram 650 illustrating the intravascular device 195 within the vessel 80 similar to that of FIGS. 6a and 6b, but at a third stage of the pulse wave when the pulse wave is essentially at the peak and the vessel boundary is essentially at its maximum expansion and the vessel wall 605 is essentially maximally stretched.

FIGS. 7a-c show schematic examples of cross-sectional views of the vessel 80 with a intravascular device 195 inside the vessel 80. FIGS. 7a-c show the cross-sectional boundary of a specific location of the vessel 80 such as at the specific location corresponding FIGS. 6a-c at three different times. For example, the diagrams 700, 720, and 740 show the cross-sectional area when the pressure wave 602 of FIGS. 6a-c is at a minimum, midway between minimum and the peak, and at essentially the peak, at the specific location designated by dashed line 604. The diagrams also show the radius 704 of the cross sections as well as the intravascular device 195 inside the cross sections. As shown, the boundary of the vessel 80 can expand, e.g., the wall can stretch and become narrower, due to pressure wave. As shown in the graphs, the cross-sectional area (i.e., the radius 704) of the vessel can increase between the diagrams 700 to 740. In particular, FIG. 7a is a schematic diagram illustrating a cross-sectional view of the vessel associated with the first stage of the pulse wave shown in Fig. 6a. FIG. 7b is a schematic diagram illustrating a cross-sectional view of the vessel associated with the second stage of the pulse wave shown in Fig. 6b. FIG. 7c is a schematic diagram illustrating a cross-sectional view of the vessel associated with the third stage of the pulse wave shown in Fig. 6c. As shown in FIGS. 7a-c, from FIG. 7a to FIG. 7c, as the pressure increases, the radius 704 increases, and the wall thickness 701, 702, 703 decreases (stretches).

FIG. 8 provides a flow diagram illustrating a method 800 of determining pulse wave velocity in a vessel. The method 800 can be performed with reference to FIGS. 1a, 1b, 2, 6a, 6b, and 6c. At step 802, a pressure is monitored within a vessel, e.g., vessel 80. The pressure can be monitored with the pressure sensor 204 shown in FIG. 1a, 1b, 2, 6a, 6b, and 6c. The pressure sensor can be part of an intravascular device 195 that is positioned inside the vessel 80. As shown in FIG. 1a, the pressure sensor 204 can communicate through the interface module 120 with the processor 140 such that the processor 140 can control the pressure monitoring of the pressure sensor 204. In an example the processor can receive pressure data associated with the monitoring of the pressure by the pressure sensor 204. In an example the interface module 120 can receive signals corresponding to pressure monitoring from the pressure sensor and can sample the pressure signals to provide the pressure data. In some examples, the steps 802 and 804 may be performed in any order, or be performed simultaneously.

At step 804 of the method 800 a wall thickness of the vessel 80 is monitored. A cross section of the vessel 80 can be monitored by an imaging element 202 shown in FIG. 1a, 1b, 2, 6a, 6b, and 6c. By monitoring a cross section of the vessel 8, the vessel wall thickness of that cross section can be monitored. In an example, the imaging element can be part of the intravascular device 195 that is positioned inside the vessel 80. In another example, the imaging element can be part of a separate intravascular device 196 inside the vessel, or the imaging element can be outside the vessel 80. As shown in FIG. 1a, the imaging element 202 can communicate through the interface module 120 with the processor 140 such that the processor 140 can control the wall thickness monitoring of the imaging element 202. In an example the processor can receive wall thickness data associated with monitoring of the wall thickness of the vessel 80 by the imaging element 202. In an example the interface module 120 can receive signals corresponding to wall thickness monitoring from the imaging element 202 and can sample the received signals to provide the wall thickness data.

Referring back to FIG. 2, the intravascular device 110 (e.g., a guidewire or catheter) can be positioned within the renal anatomy. In some instances, prior to insertion of the intravascular device 110, a guidewire or guide catheter may be introduced into the arterial vasculature of a patient using standard percutaneous techniques. Once the guidewire or guide catheter is positioned within the target blood vessel, which is the left renal artery 81 in the illustrated embodiment of FIG. 2, the intravascular device 110 may be introduced into the arterial vasculature of a patient over the guidewire or through the guiding catheter and advanced to the area of interest. In the alternative, the intravascular device 110 may be coupled to the guidewire or guide catheter external to the patient and both the guidewire/guide catheter and the intravascular device 110 may be introduced into the patient and advanced to an area of interest simultaneously. Additionally, the user may utilize external imaging, such as, byway of non-limiting example, fluoroscopy, ultrasound, CT, or MRI, to aid in the guidance and positioning of the intravascular device 110 within the patient's vasculature. In some instances, the intravascular device 110 is introduced without use of a guidewire or guide catheter.

At step 806 of the method 800 a pressure data associated with the monitoring of the pressure within the vessel 80 is received. Also, a wall thickness data associated with monitoring of the wall thickness of the vessel 80 is received. As described above, the interface module 120 can receive both signals corresponding to pressure monitoring from the pressure sensor 204 and signals corresponding to the wall thickness monitoring from the imaging element 202. In an example, the interface module 120 can sample the received signals and provide the wall thickness data and the pressure data to the processor 140.

At step 808 of the method 800 the pulse wave velocity of a fluid within the vessel 80 is determined based on the pressure data within the vessel 80 and the wall thickness data of the vessel 80. In an example, the imaging element 202 can measure a wall thickness of the vessel at a specific cross section of the vessel 80 and the pressure sensor 204 can measure the pressure inside the vessel at essentially the same location. As described above and shown in FIGS. 1a, 6a, 6b, and 6c, the pressure sensor 204 and imaging element 202, although on the same intravascular device, can have a separation D. Therefore, at each instance of time, the pressure sensor 204 and imaging element 202 may not generate the pressure signal and imaging element signal of exactly the same location of the vessel 80. As described before, the signals received from the pressure sensor 204 and imaging element 202 can be sampled by the interface module 120. In an example, the interface module 120 can synchronize the sampled wall thickness data and the pressure data and can generate wall thickness data and pressure data corresponding to a same instance of time. Alternatively, the processor can use interpolation on the wall thickness data and the pressure data to find the wall thickness data and the pressure data corresponding to a same time at an essentially a same location. As an example, the processor 140 can use the equation (4) to determine the pulse wave velocity. As described, the processor can determine a change in the wall thickness data and a change in pressure data and use the equation (4) to calculate the pulse wave velocity.

In some embodiments, before initializing the application of method 800, the user and/or the processor 140 may utilize the intravascular device 195 to do baseline measurements of various cardiovascular characteristics of the vessel, including by way of non-limiting example, a vessel lumen volume. For example, by moving the intravascular device 195 and its pressure sensor 204 and imaging element 202 through the vessel and sampling the pressure and wall thickness of the vessel at one or more location for at least the duration of a pulse and create temporal and spatial correlation data and to use this data to find the wall thickness data and the pressure data corresponding to a same time at a an essentially a same location. Alternatively, based on a first pulse wave velocity measurement in the vessel 80 and also based on the distance between pressure sensor 204 and imaging element 202, the time difference for the pressure wave to travel between pressure sensor 204 and imaging element 202 can be estimated. Using this estimated time difference, the sampled pressure and wall thickness data can additionally be synchronized in time for essentially the same location inside the vessel 80 and a new (e.g., more accurate) pulse wave velocity can be calculated. In an example, based on the sampling rate of the pressure and wall thickness data, the above procedure may be repeated.

In some embodiments, the method 800 optionally includes determining a therapy recommendation based on the PWV. In some instances, a clinician determines the therapy recommendation based on the computed PWV and/or other patient data. In some embodiments, the processing system evaluates the PWV and/or other patient data to determine the therapy recommendation. In such instances, the method 800 includes outputting a visual representation of the therapy recommendation. For example, the processing system can output display data associated with the graphical representation to a display device. The can be a textual indication, such as "Poor," "Fair," "Good," and/or other suitable words may communicate the predicted benefit associated with therapy for the particular patient. In other instances, a numerical score, color coding, and/or other graphics representative of the therapy recommendation can be output to the display. The therapy can be renal denervation in some instances. The method 800 can additionally include classifying, based on the PWV, one or more patients into groups corresponding to respective degrees of predicted therapeutic benefit as a result of the renal denervation. The method 800 can also include the processing system outputting a graphical representation of the classifying step to the display device.

It should be appreciated that the imaging device described herein can utilize backscattered data (or a transformation thereof) based on electromagnetic radiation (e.g., light waves in non-visible ranges such as Optical Coherence Tomography, X-Ray CT, etc.) to render images of any tissue type or composition (not limited to vasculature, but including other human as well as non-human structures). Such imaging techniques are within the spirit and scope of the present disclosure.

Persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. For example, the intravascular device may be utilized anywhere with a patient's vasculature, both arterial and venous, having an indication for thermal neuromodulation. It is understood that such variations may be made to the foregoing without departing from the scope of the present disclosure. Accordingly, it is appropriate that the appended claims be construed broadly and in a manner consistent with the present disclosure.

## Claims

1. An apparatus for pulse wave velocity (PWV) determination in a vessel, the apparatus comprising:

   an intravascular device including a flexible elongate member that has a proximal portion and a distal portion, wherein at least the distal portion of the intravascular device is configured to be positioned within the vessel, and wherein a pressure sensor is coupled to the distal portion of the flexible elongate member and is configured to monitor a pressure within the vessel;

   at least one imaging element positioned within the vessel and configured to monitor a wall thickness of the vessel; and

   a processing system in communication with the pressure sensor and the at least one imaging element, the processing system configured to:

   receive pressure data associated with the monitoring of the pressure within the vessel by the pressure sensor;

   receive wall thickness data associated with monitoring of the wall thickness of the vessel by the at least one imaging element; and

   determine a pulse wave velocity of fluid within the vessel based on the pressure data and the wall thickness data.

2. The apparatus of claim 1, wherein the pulse wave velocity is determined as $\sqrt{\dfrac{dP}{2\rho} \cdot \dfrac{h}{-dh}}$, wherein $dP$ is a change in pressure based at least on the pressure data, $h$ is a thickness of the vessel wall based at least on the wall thickness data, $dh$ is a change in thickness of the vessel wall based at least on the wall thickness data, and $\rho$ is a density of a fluid within the vessel.

3. The apparatus of claim 1, wherein the vessel comprises a renal artery.

4. The apparatus of claim 3, wherein the processing system is further configured to:

   determine a renal denervation therapy recommendation based on the pulse wave velocity.

5. The apparatus of claim 3, wherein the processing system is further configured to:

   classify a patient based on a predicted therapeutic benefit of renal denervation using the pulse wave velocity.

6. The apparatus of claim 1, wherein the at least one imaging element is coupled to the distal portion of the flexible elongate member of the intravascular device.

7. The apparatus of claim 1, wherein the at least one imaging element is coupled to an intravascular probe that is separate from the intravascular device.

**8.** The apparatus of claim 7, wherein the intravascular device comprises a guide wire, and wherein the intravascular probe comprises a catheter.

**9.** The apparatus of claim 1, wherein the at least one imaging element comprises an optical coherence tomography imaging element.

**10.** A method of determining pulse wave velocity (PWV) in a vessel, comprising:

monitoring a pressure within the vessel with a pressure sensor positioned within the vessel;
monitoring a wall thickness of the vessel by at least one imaging element positioned within the vessel;
receiving pressure data associated with the monitoring of the pressure within the vessel by the pressure sensor;
receiving wall thickness data associated with the monitoring of the wall thickness of the vessel; and
determining the pulse wave velocity of a fluid within the vessel based on the received pressure data and the received wall thickness data.

**11.** The method of claim 10, wherein the pressure sensor is coupled to a first intravascular device positioned within the vessel and the at least one imaging element is coupled to a second intravascular device positioned within the vessel.

**12.** The method of claim 10, wherein the pulse wave velocity is determined as $\sqrt{\dfrac{dP}{2\rho} \cdot \dfrac{h}{-dh}}$ , and wherein *dP* is a change in pressure based at least on the pressure data, *h* is a thickness of the vessel wall based at least on the wall thickness data, *dh* is a change in thickness of the vessel wall based at least on the wall thickness data, and $\rho$ is a density of a fluid within the vessel.

**13.** The method of claim 10, wherein the vessel is a renal artery.

**14.** The method of claim 13, further comprising:

determining a renal denervation therapy recommendation based on the pulse wave velocity.

**15.** The method of claim 13, further comprising:

classifying a patient based on a predicted therapeutic benefit of renal denervation using the pulse wave velocity.

DISPLAY
160

Processing System
130

PROCESSOR
140

MEMORY
150

INTERFACE
MODULE
120

100

110

202

204

80

170

82

Fig. 1a

Fig. 1b

Fig. 2

Fig. 3

Fig. 4a

*Fig. 4b*

Fig. 5a.

Fig. 5b

Fig. 6a

Fig. 6b

Fig. 6c

701

700

110

605

r

704

**FIG. 7a**

720

702

110

605

r

704

**FIG. 7b**

740

703

110

605

r

704

**FIG. 7c**

800 ⟍

```
┌─────────────────────────────────────────────────────────┐
│  Monitor a pressure within the vessel with a pressure    │
│  sensor positioned within the vessel                     │
│  802                                                     │
└─────────────────────────────────────────────────────────┘
                          │
                          ▼
┌─────────────────────────────────────────────────────────┐
│  Monitor a wall thickness of the vessel                  │
│  804                                                     │
└─────────────────────────────────────────────────────────┘
                          │
                          ▼
┌─────────────────────────────────────────────────────────┐
│  Receive pressure data associated with the monitoring    │
│  of the pressure within the vessel and wall thickness    │
│  data associated with monitoring of the wall thickness   │
│  806   of the vessel                                     │
└─────────────────────────────────────────────────────────┘
                          │
                          ▼
┌─────────────────────────────────────────────────────────┐
│  Determine the pulse wave velocity of a fluid within     │
│  the vessel based on the pressure data within the vessel │
│  808   and wall thickness data of the vessel.            │
└─────────────────────────────────────────────────────────┘
```

FIG. 8

Europäisches
Patentamt

European
Patent Office

Office européen
des brevets

# EUROPEAN SEARCH REPORT

Application Number

EP 16 17 6925

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/34724 A2 (FLORENCE MEDICAL LTD [IL]; DGANY ELHANAN [IL]; NOSKOWICZ SIMON HENRI []) 15 July 1999 (1999-07-15) | 1-3,6-13 | INV.<br>A61B5/00<br>A61B8/12 |
| Y | * page 20, line 5 - line 17 *<br>* page 31, line 5 - line 11 *<br>* page 24, line 15 - page 26, line 25 *<br>* page 28, line 9 - line 17 *<br>* figures 1-3, 8 *<br>----- | 4,5,14,15 | A61B5/02<br>A61B5/021<br>A61B5/0215 |
| Y | P LURZ ET AL: "Aortic pulse wave velocity as a marker for arterial stiffness predicts outcome of renal sympathetic denervation and remains unaffected by the intervention",<br>EUROPEAN HEART JOURNAL,<br>vol. 36, no. Suppl. 1,<br>1 August 2015 (2015-08-01), page 387,<br>XP055329401,<br>GB<br>ISSN: 0195-668X, DOI:<br>10.1093/eurheartj/ehv399<br>* abstract *<br>----- | 4,5,14,15 | |
| A | US 2010/113949 A1 (SATHYANARAYANA SHASHIDHAR [US]) 6 May 2010 (2010-05-06)<br>* paragraph [0030] - paragraph [0036] *<br>* paragraph [0041] - paragraph [0043] *<br>* figures 4A-D,6A-B *<br>* paragraph [0025] *<br>----- | 1-15 | TECHNICAL FIELDS SEARCHED (IPC)<br><br>A61B<br>G06T |
| A | US 2014/012133 A1 (SVERDLIK ARIEL [IL] ET AL) 9 January 2014 (2014-01-09)<br>* paragraph [0160] - paragraph [0172] *<br>----- | 1-15 | |

The present search report has been drawn up for all claims

| Place of search | Date of completion of the search | Examiner |
|---|---|---|
| The Hague | 21 December 2016 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 17 6925

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2016

| Patent document cited in search report | | Publication date | Patent family member(s) | | Publication date |
|---|---|---|---|---|---|
| WO 9934724 | A2 | 15-07-1999 | AU | 1781699 A | 26-07-1999 |
| | | | WO | 9934724 A2 | 15-07-1999 |
| US 2010113949 | A1 | 06-05-2010 | US | 2006058653 A1 | 16-03-2006 |
| | | | US | 2010113949 A1 | 06-05-2010 |
| | | | WO | 2006023924 A1 | 02-03-2006 |
| US 2014012133 | A1 | 09-01-2014 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8108030 B, Castella
- US 20110152771 A, Milner
- US 20100220334 A, Condit
- US 20090043191 A, Castella
- US 20080291463 A, Milner
- US 20080180683 A, Kemp, N.
- US 5321501 A

- US 7999938 B
- US 7995210 B
- US 7787127 B
- US 7783337 B
- US 6134003 A
- US 6421164 B